(19) Europäisches Patentamt
European Patent Office
Office européen des brevets

(11) **EP 4 509 161 A1**

(12) **EUROPEAN PATENT APPLICATION**
published in accordance with Art. 153(4) EPC

(43) Date of publication:
**19.02.2025 Bulletin 2025/08**

(21) Application number: 23788335.0

(22) Date of filing: **11.04.2023**

(51) International Patent Classification (IPC):
**A61M 25/10** (2013.01)    **A61B 17/12** (2006.01)
**A61B 5/026** (2006.01)

(52) Cooperative Patent Classification (CPC):
**A61B 5/026; A61B 17/12; A61M 25/10**

(86) International application number:
**PCT/JP2023/014708**

(87) International publication number:
**WO 2023/199917 (19.10.2023 Gazette 2023/42)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **11.04.2022 JP 2022064939**

(71) Applicant: **Keio University
Tokyo, 108-8345 (JP)**

(72) Inventors:
• **OGAWA Emiyu
Sagamihara-shi, Kanagawa 252-0373 (JP)**
• **MARUHASHI Takaaki
Sagamihara-shi, Kanagawa 252-0373 (JP)**
• **KAWAKAMI Sota
Sagamihara-shi, Kanagawa 252-0373 (JP)**
• **SAMEJIMA Yuya
Sagamihara-shi, Kanagawa 252-0373 (JP)**

(74) Representative: **D Young & Co LLP
3 Noble Street
London EC2V 7BQ (GB)**

(54) **DEVICE FOR MONITORING BLOOD BLOCKAGE RATE BY AORTIC BLOCKAGE BALLOON**

(57)    To provide a device for monitoring a blood flow at the time when the blood flow is stopped using an aortic blockage balloon to thereby prevent complete blockage of the blood flow and prevent outbreak of complications. A balloon catheter device comprising: a balloon catheter including a catheter tube and an aortic blockage balloon provided at a distal end portion of the catheter tube; one or more optical fibers housed in the catheter tube; one or more light irradiation devices disposed in the balloon and coupled to the optical fibers and one or more light detection parts that detect backscattered light of radiated light; and one or more light sources that generate light to be radiated, wherein the balloon catheter device radiates, from the aortic blockage balloon into an aorta, light having a wavelength that can be absorbed by a substance present in blood, detects backscattered light from an inside of a blood vessel, and monitors a state of a blood flow between the aortic blockage balloon and a blood vessel wall over time from intensity of the detected light.

Fig. 2

## Description

Technical Field

The present invention relates to a device for monitoring a blood blockage rate by an aortic blockage balloon and a monitoring method using the device.

Background Art

As a method of an aortic blockage technique at the time of damage by a trauma, open aortic cross clamp (RTACC) or open aortic compression has been performed. However, excessive invasion has been a problem.

As a method of solving the problem of the excessive invasion, a method of blocking a blood flow of an aorta using an aortic blockage balloon (REBOA: Resuscitative Endovascular Balloon Occlusion of the Aorta) has been developed. In the method, a balloon catheter is percutaneously inserted from a femoral artery and the balloon is inflated in an aorta to block the blood flow of the aorta. The method is a low invasive hemostasis method and has also been capable of preventing low body temperature.

In the REBOA, it has been possible to prevent complications due to ischemia in the extremities by adjusting a balloon diameter with a liquid injection amount into the balloon, making a blocking area of the aorta to be partial (partial-REBOA), and maintaining a blood flow on the peripheral side.

However, a blood vessel diameter of the artery has easily changed according to an intravascular volume. Even if there has been no change in a balloon volume, in some case, the blood flow has been completely blocked (over-inflation) and complications have developed (see Non Patent Literature 1).

Citation List

Non Patent Literature

Non Patent Literature 1: Jonker FH et al., Eur J Vasc Endovasc Surg 2010; 40:564-71

Summary of Invention

Technical Problem

In order to stop bleeding from a trauma, an aortic blockage balloon has been used. However, since the blood vessel diameter of the aorta has easily changed, in some case, the blood flow has been completely blocked (Over-inflation) and complications have developed.

An object of the present invention is to provide a device for monitoring a blood flow at the time when the blood flow is stopped using an aortic blockage balloon to thereby prevent complete blockage of the blood flow and prevent outbreak of complications.

Solution to Problem

The present inventor has found that, by radiating, from a balloon into a blood vessel, light having a wavelength absorbed by a substance present in the blood vessel and detecting backscattered light from the inside of the blood vessel, it is possible to monitor presence or absence of a blood flow between the blood vessel and the balloon using the backscattered light and it is possible to detect unexpected Over-inflation and prevent complications.

Further, the inventor has also found that, by using lights having a plurality of wavelengths with different optical penetration depths as light to be radiated, it is possible to predict a blood flow rate.

In the present invention, in the REBOA, blood flow blockage (over-inflation) is detected by monitoring the blood flow between the blood vessel and the balloon using the backscattered light. As a result, it is possible to prevent complications. That is, in the present invention, it is possible to solve the problems in the REBOA.

The present invention is as described below.

[1] A balloon catheter comprising:

a balloon catheter including a catheter tube and a balloon provided at a distal end portion of the catheter tube;
an optical fiber housed in the catheter tube;
a light irradiation part disposed in the balloon and coupled to the optical fiber and a light detection part that detects radiated light; and

a light source that generates light to be radiated, wherein

the balloon catheter makes it possible to radiate, from the balloon, light having a wavelength that can be absorbed by a substance present in blood, detect scattered light from an inside of a blood vessel, and monitor a state of a blood flow between the balloon and a blood vessel wall over time from intensity of the detected light.

[2] The balloon catheter of [1], comprising an arithmetic operation part that determines presence or absence of a blood flow based on the intensity of the scattered light.

[3] The balloon catheter of [2], comprising a display part for displaying a state of the blood flow analyzed by the arithmetic operation part.

[4] The balloon catheter of any one of [1] to [3], wherein a wavelength of the light to be radiated is 400 to 500 nm.

[5] The balloon catheter of any one of [1] to [4], wherein the balloon catheter radiates, from the balloon, the light having the wavelength that can be absorbed by the substance present in the blood, the light being lights having a plurality of wavelengths with different optical penetration depths, detects scattered lights from the inside of the blood vessel of the lights having the respective wavelengths, and monitors a blood flow rate between the balloon and the blood vessel wall from intensities of the scattered lights of the lights having the respective wavelengths.

[6] The balloon catheter of [5], wherein the balloon catheter defines a function represented by Expression (1) or (2) described below, performs fitting using an elapsed time from a balloon inflation start at a time when backscattered light is detected and data of backscattered light intensity, determines a determination coefficient $\alpha$,

$$T = -\frac{a}{\alpha p(p-1)} log \frac{\frac{P}{1-P}}{\frac{p}{1-p}} + t \qquad (1)$$

T: time

$\alpha$: value determined for each wavelength

P: any backscattered light intensity

p: actual backscattered light intensity closest to $\frac{a}{2}$

*t*: time at a time when *P=p*

or

$$T(P) = \frac{log \frac{P}{1-P}}{b} + c \qquad (2)$$

*T(P)*: time

P: any backscattered light intensity

b: coefficient determined for each wavelength

C: coefficient determined for each wavelength

uses the calculated determination coefficient and substitutes, in Expression (1) or (2) described above, backscattered light intensity detected by radiating light (green) having a wavelength of 560 nm or light having a wavelength of 650 nm to thereby calculate an elapsed time after the inflation start,

estimates a balloon diameter from the obtained time after the inflation start, and

calculates a blood layer thickness according to an expression described below [blood layer thickness=blood vessel diameter (inner diameter)-balloon diameter].

[7] The balloon catheter of [5] or [6], wherein the lights with the different optical penetration depths are lights having two kinds, three kinds, four kinds, five kinds, or six kinds of wavelengths among lights having wavelengths such as near infrared light, infrared light, 380 to 430 nm light (violet), 430 nm to 490 nm light (blue), 490 nm to 550 nm light (green), 550 nm to 590 nm light (yellow), 590 nm to 640 nm light (orange), and 640 to 770 nm light (red).

[8] The balloon catheter of [5] or [6], wherein the lights with the different optical penetration depths are at least two kinds among light having a wavelength of 475 nm, light having a wavelength of 542 nm, and light having a wavelength of 438 nm.

[9] The balloon catheter of [5] or [6], wherein the lights with the different optical penetration depths are two kinds of green light and red light.

[10] The balloon catheter of [5] or [6], wherein the lights with the different optical penetration depths are two kinds of green light having a wavelength of 560 nm and red light having a wavelength of 650 nm.

[11] The balloon catheter of any one of [1] to [10], wherein the balloon catheter detects the scattered light in back.

[12] The balloon catheter of any one of [1] to [11], wherein the catheter is a blood vessel blockage or aortic blockage catheter.

The present specification includes the disclosed content of Japanese Patent Application No. 2022-64939 that is a basis of the priority of the present application.

Advantageous Effects of Invention

By using the balloon catheter of the present invention, it is possible to monitor presence or absence of a blood flow between the balloon and the blood vessel wall during aortic blockage by the balloon or a blood flow rate and it is possible to prevent outbreak of complications due to complete blockage of the aorta by adjusting a balloon diameter with the blood flow rate as an indicator.

Brief Description of Drawings

[Figure 1] Figure 1 is a diagram illustrating structure of a balloon catheter of a device that monitors a blood blockage rate by an aortic blockage balloon.

[Figure 2] Figure 2 is a diagram illustrating structure of the device that monitors the blood blockage rate by the aortic blockage balloon.

[Figure 3] Figure 3 is a diagram illustrating backscattered light intensity in the device that monitors the blood blockage rate by the aortic blockage balloon. Figure 3A is a diagram illustrating backscattered light intensity at wavelengths in 5 and 10 (v/v)% intralipid solutions of an inflated balloon (n=3) and Figure 3B is a diagram illustrating a time from an inflation start to a backscattered light intensity differential maximum value due to a difference in optical penetration depth (*ex vivo*, mean±SD, n=4).

[Figure 4] Figure 4 is a diagram illustrating a backscattered light intensity change involving *in vivo* inflation and deflation of a REBOA balloon.

[Figure 5] Figure 5 is a diagram illustrating intensities (unit AU: Arbitrary Unit) of backscattered lights of lights having wavelengths and a balloon diameter in a time (the horizontal axis) from an inflation start in the case in which 560 nm light (green) and 650 nm light (red) are used.

[Figure 6] Figure 6 is a diagram illustrating intensities (unit AU: Arbitrary Unit) of backscattered lights of lights having wavelengths and a balloon diameter in a time (the horizontal axis) from an inflation start in the case in which 395 nm light (violet) and the 560 nm light (green) are used (Figure 6).

[Figure 7] Figure 7 is a diagram illustrating whether backscattered lights measured using the 395 nm light (violet), the 560 nm light (green), and the 650 nm light (red) at the time when the blood layer thickness is large to small have exceeded a threshold (indicated by "O") or have not exceeded the threshold (indicated by " × ").

[Figure 8] Figure 8 is a diagram illustrating an overview of a method of measuring blood flow velocity with light.

[Figure 9] Figure 9 is a sectional view of a blood vessel into which a balloon is inserted and a diagram illustrating a balloon diameter and a blood layer thickness.

[Figure 10] Figure 10 is a diagram illustrating backscattered light intensity changes of red light and green light in an *in vivo* porcine aorta.

Description of Embodiments

The present invention is described in detail below.

1. Device for monitoring a blood blockage rate by a blood vessel blockage balloon

The present invention is a device for, in blood vessel blockage using a balloon catheter, irradiating, from a balloon, a blood vessel wall with light having a wavelength absorbed by a substance present in a blood vessel and detecting backscattered light from the inside of the blood vessel to thereby monitor a blood flow between the blood vessel and the balloon using the backscattered light and a monitoring method using the device. "Monitoring a blood flow between the blood vessel and the balloon" can also be referred to as "monitoring a blood blocking rate by the balloon".

A device used in the present invention includes:

a balloon catheter including a catheter tube and a blood vessel blockage balloon provided at the distal end portion of the catheter tube;
one or more optical fibers housed in the catheter tube;
one or more light irradiation parts disposed in the balloon and coupled to the optical fibers and one or more light detection parts that detect backscattered light of radiated light; and
one or more light sources (light generation parts) that generate light to be radiated.

The device of the present invention is also simply called a balloon catheter.

When light having a wavelength absorbed by a substance present in a blood vessel is radiated from a balloon into the blood vessel, if blood is present between the balloon and a blood vessel wall, the radiated light is partially absorbed and the intensity of backscattered light decreases. The intensity of the backscattered light is measured and presence or absence of a blood flow in the blood vessel and a blood flow rate are monitored.

Using the device, it is possible to radiate, from the blood vessel blockage balloon into the blood vessel, light having a wavelength that can be absorbed by a substance present in blood, detect backscattered light from the inside of the blood vessel, and monitor presence or absence of a blood flow between the blood vessel blockage balloon and the blood vessel wall over time from the intensity of the detected light.

The "blood vessel" includes any blood vessel if the balloon catheter can be inserted into the blood vessel and is preferably an aorta.

The "catheter" refers to a thin tube that can be inserted into the blood vessel. As the balloon catheter of the present invention, a catheter with a balloon usually used in a blood vessel endoscope or the like can be used. The diameter and the like of the balloon catheter are not limited. The balloon catheter is used to press a blood vessel wall of an aorta or the like with appropriate pressure and block a blood flow at the time of bleeding. The balloon catheter includes a catheter tube balloon that is also called a shaft. Means for inflating the balloon is not particularly limited but can be achieved by supplying appropriate liquid and gas into the balloon. The device of the present invention includes liquid feeding and absorbing means or air feeding and absorbing means for inflating and deflating the balloon of the balloon catheter. The liquid feeding and absorbing means or the air feeding and absorbing means is provided in the catheter tube as a lumen. The balloon is attached near a distal end portion of the catheter. The catheter tube may have a double structure including an outer tube and an inner tube. In this case, the lumen is formed between the two tubes. Pressure of the balloon at the inflation time pressing the blood vessel wall is desirably between 0.2 kg/cm$^2$ and 1 kg/cm$^2$.

The balloon that can be used for blood flow blockage is referred to as an occlusion balloon or a block balloon as well.

As the material of the balloon, nylon (registered trademark), polyvinyl chloride, polyethylene, polyurethane, polyether block amide copolymer, olefin polymers such as polyethylene terephthalate and polypropylene, or copolymers including combinations of these materials can be used. Silicone rubber, latex rubber, and the like can also be used. The size of the balloon is preferably approximately 10 to 50 mm in an inflated diameter and approximately 5 to 100 mm in length in the longitudinal direction.

The catheter distal end of the present invention may adopt a freely bending structure. For this purpose, for example, a tension wire can be disposed in the catheter and the distal end portion can be bent by towing operation of the tension wire. Further, the distal end portion may be bent in advance to match the shape of a site to be treated. The device of the present invention may include a guide sheath or a guide wire for causing the catheter to be inserted and advance to a target site. The size of the catheter is preferably 6 to 10 Fr. The catheter has to be inserted into a body from a femoral artery or a brachial artery according to the rule. Examples of the material of the catheter tube include polyolefin such as polyethylene, polypropylene, ethylene-propylene copolymer, and ethylene-vinyl acetate copolymer, and thermoplastic resin such as polyvinyl chloride, polyamide, polyimide, polyethylene terephthalate, polyamide elastomer, polyester, polyester elastomer, and polyurethane.

Means for transmitting light to a light irradiation part in the balloon includes a light irradiation part and an optical fiber that transmits light from a light source to the light irradiation part, the light irradiation part and the optical fiber being located near the distal end portion of the catheter. In the present specification, "near the distal end portion" means a portion close to the end portion on the opposite side of the end portion (the proximal end portion) coupled to a high-intensity pulse light generation part and indicates the distal end portion and a portion approximately several ten centimeters from the distal end portion.

Further, the device of the present invention includes the light source (a light generation part). The light source can generate light having any wavelength and radiates the generated light from the light irradiation part in the balloon into the blood vessel toward a blood vessel wall through the optical fiber coupled to the light source. The device of the present invention may include one or more light irradiation parts such that lights having a plurality of wavelengths can be simultaneously radiated. The light irradiation part is not limited but examples of the light irradiation part include an LED generation part.

The optical fiber is housed in the catheter tube and is coupled to the light source at one end of the optical fiber. An appropriate device for laser light irradiation such as a lens may be provided at the optical fiber distal end. As the optical fiber used in the present invention, optical fibers having a wide variety of diameters ranging from an extremely thin optical fiber having a diameter of approximately 0.05 to 0.6 mm to an optical fiber having visible thickness.

The device of the present invention further includes a light detection part for detecting backscattered light radiated into the blood vessel from the light irradiation part and reflected and scattered in the blood vessel. The device of the present invention may include one or more light detection parts such that lights having a plurality of wavelengths can be simultaneously detected.

The device for monitoring the blood blockage rate of the present invention further includes an arithmetic operation part for determining presence or absence of a blood flow based on the intensity of backscattered light detected by the device and a display part for displaying the presence or absence of the blood flow analyzed by the arithmetic operation part.

Examples of the substance present in the blood vessel include hemoglobin. Hemoglobin absorbs light having a wavelength of 400 to 500 nm. Therefore, the wavelength of the light radiated from the light source is 400 to 500 nm.

Further, by radiating lights having a plurality of wavelengths with different optical penetration depths and detecting backscattered lights from the inside of the blood vessel of the lights having the respective wavelengths, a blood flow rate between the blood vessel blockage balloon and the blood vessel wall can be monitored from the intensities of the backscattered light of the lights having the respective wavelengths. Examples of the lights having the plurality of wavelengths include 475 nm light (cyan), 542 nm light (Green), and 438 nm light (Blue). In this case, in order to transmit, radiate, and receive the lights having the plurality of wavelengths, the device of the present invention may include a plurality of optical fibers, a plurality of light irradiation parts, and a plurality of light detection parts (light reception parts).

2. Backscattered light detection method using the device for monitoring the blood blockage rate by the blood vessel blockage balloon

Monitor light having a wavelength absorbed by the substance present in the blood vessel has to be radiated from the light irradiation part disposed in the balloon and backscattered light of the radiated light has to be detected. Here, the backscattered light refers to the light radiated from the light irradiation part near the distal end of the optical fiber being absorbed and scattered in the blood vessel and returning to the fiber again. The returning backscattered light is weak when a blood flow is present in the blood vessel and the returning backscattered light is strong when a blood flow is absent in the blood vessel. As described above, examples of the substance present in the blood vessel include a substance in blood. Hemoglobin is particularly preferable. Hemoglobin is chromoprotein and absorbs light having a specific wavelength. Therefore, since light absorption and scattering properties are different depending on an amount of hemoglobin present in the blood vessel, by detecting the backscattered light, it is possible to discriminate whether a blood flow is present in a part irradiated with light.

As the monitor light, light having a wavelength of 400 nm to 500 nm has to be used. The maximum of a wavelength of light absorbed by hemoglobin is near 400 or 550 nm. Even if the light deviates from this wavelength, since the light can be absorbed by hemoglobin, which is chromoprotein, the light can be adopted as the monitor light used in the device of the present invention. Light intensity may be small and feeble light with output of 0.01 mW to 1 mW has to be used. The monitor light is generated by a light source on the outside, transmitted through a fiber for monitor light transmission, and radiated from the fiber distal end. The backscattered light is made incident on a fiber for transmission that has radiated the monitor light and reversely travels in the fiber and returns. For detection of the backscattered light, a detector (a light detection part) for monitoring the backscattered light has to be coupled to the fiber on which the backscattered light is made incident and through which the backscattered light returns. A route of the light returning through the optical fiber has to be changed by providing a beam splitter halfway in the fiber. Further, light having a desired wavelength has to be selected through an appropriate bandpass filter and guided to a scattered light detector. The scattered light detector is not limited if the scattered light detector can detect light. For example, a silicon photodiode can be used.

Further, by radiating lights having a plurality of wavelengths with different optical penetration depths as the monitor light and detecting backscattered lights from the inside of the blood vessel of the light having the respective wavelengths, a blood flow rate between the blood vessel blockage balloon and the blood vessel wall can be monitored from the intensities of the backscattered lights of the lights having the respective wavelengths. As the lights having the plurality of wavelengths, for example, lights having a plurality of wavelengths, for example, light having wavelengths of two kinds, three kinds, four kinds, five kinds, or six kinds among 380 to 430 nm light (violet), 430 nm to 490 nm light (blue), 490 nm to 550 nm light (green), 550 nm to 590 nm light (yellow), 590 nm to 640 nm light (orange), and 640 to 770 nm light (red) have to be used. Examples of a combination of lights to be used include cyan light, green light, and blue light, violet light, green light, and red light, or green light and red light. When indicated by specific wavelengths, examples of the combination of the wavelengths of the light to be used include near infrared light, infrared light, or lights having wavelengths near the near infrared light and the infrared light. For example, examples of the combination of the wavelengths of the lights to be used include the 475 nm light (cyan), the 542 nm light (green), and the 438 nm light (blue), the 395 nm light (violet), the 560 nm light (green), and the 650 nm light (red), or the 560 nm light (green) and the 650 nm light (red). The lights having the plurality of wavelengths have to be transmitted, radiated, and received. As described below, it is possible to estimate a blood layer thickness, measure blood flow velocity, and subsequently measure a blood flow rate.

(1) Estimation of a blood layer thickness

A blood absorption coefficient is different depending on a wavelength of light to be used and a difference occurs in optical penetration depth. Hemoglobin included in blood has a peak of an absorption coefficient in wavelengths of blue to green. The absorption coefficient is small in a red wavelength. Light having a wavelength with a small absorption coefficient is less easily absorbed in the blood by hemoglobin in the blood, and therefore optical penetration depth in the blood is large. When the balloon is inflated in the blood and the monitor light is radiated from the light irradiation part in the balloon, the light having the wavelength with the small absorption coefficient is less easily absorbed by a substance in the blood, the optical penetration depth increases, and the backscattered light intensity increases, and the light having a wavelength with a large absorption coefficient is easily absorbed by the substance in the blood, the optical penetration depth decreases, and the backscattered light intensity decreases.

It is possible to inflate the blood vessel blockage balloon in the blood vessel, measure backscattered light intensity at the time when lights having wavelengths are used in a process of the inflation, use, as a threshold, backscattered light intensity of 60 to 80%, preferably 65 to 75%, and more preferably 70% with respect to maximum backscattered light intensity of the lights having the wavelengths, determine whether measurement values in the case in which the lights having the wavelengths are used exceed the threshold, and estimate a blood layer thickness from a pattern of the threshold determination. The blood layer thickness can be estimated as, for example, being larger than x1 mm (>x1 mm), x2 to x1 mm (larger than x2 mm and equal to or smaller than x1 mm), x3 to x2 mm (larger than x3 mm and equal to or smaller than x2 mm), and equal to or smaller than x3 mm (<0.1 mm). Here, x1, x2, x3, and x4 are any numerical values of 0.05 to 10 mm, preferably 0.05 to 5 mm, and more preferably 0.1 to 1 mm.

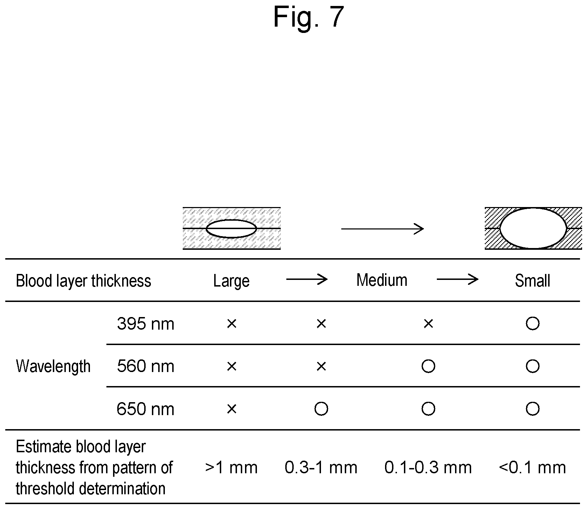

For example, the blood layer thickness can be estimated as indicated by an example 2 and Figure 7 described below. Figure 7 illustrates whether backscattered lights measured using the 395 nm light (violet), the 560 nm light (green), and the 650 nm light (red) at the time when the blood layer thickness is large to small have exceeded the threshold (indicated by "O") or have not exceeded the threshold (indicated by "×"). The blood layer thickness can be estimated as >1 mm, 0.3 to 1 mm, 0.1 to 0.3 mm, or <0.1 mm from a pattern of the threshold determination.

Further, the blood layer thickness can be estimated by, for example, a method indicated by an example 3 described below. That is, a function represented by Expression (1) or (2) described below is defined, fitting is performed using an elapsed time from a balloon inflation start at the time when backscattered light is detected and data of backscattered light intensity, and the determination coefficient $\alpha$ is determined.

$$T = -\frac{a}{\alpha p(p-1)} \log \frac{\frac{P}{1-P}}{\frac{p}{1-p}} + t \qquad (1)$$

$T$: time
$\alpha$: value determined for each wavelength
$P$: any backscattered light intensity

$p$: actual backscattered light intensity closest to $\dfrac{a}{2}$
t: time at the time when $P=p$

or

$$T(P) = \frac{log \dfrac{P}{1-P}}{b} + c \qquad (2)$$

*T(P)*: time

P: any backscattered light intensity
b: coefficient determined for each wavelength
C: coefficient determined for each wavelength

An elapsed time after an inflation start can be calculated by using the calculated determination coefficient and substituting, in Expression (1) or (2) described above, backscattered light intensity detected by radiating light (green) having a wavelength of 560 nm or light having a wavelength of 650 nm. A balloon diameter can be seen from the obtained elapsed time after the inflation start. The blood layer thickness can be calculated from an expression [blood layer thickness=blood vessel diameter (inner diameter)-balloon diameter] described below. Note that the balloon diameter can be learned from a time after the inflation start based on a measurement value. Expressions (1) and (2) described above are an example. Other expressions can be used.

(2) Measurement of blood flow velocity

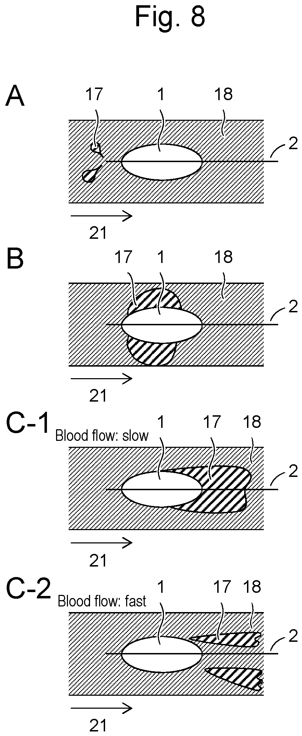

Blood flow velocity can be measured by various methods. For example, the blood flow velocity can be measured using light. A measurement method using light is illustrated in Figure 8. When the blood flow velocity is measured with light, the balloon has a lumen structure that can flush saline or the like from the distal end thereof (Figure 8A). Even if the saline is flushed without an inflation state of the balloon being changed, blood around the balloon is temporarily eliminated and the backscattered light intensity changes (Figure 8B). A time required from when the saline around the balloon is flushed until the backscattered light returns to an original value is different depending on the blood flow velocity. The blood flow velocity (m/s) has to be estimated from return velocity of the backscattered light (Figure 8C).

(3) Measurement of a blood amount

A blood amount per unit time of blood flowing in the blood vessel can be calculated from the values estimated and measured in (1) and (2). For example, the blood amount can be calculated by an expression described below.

Blood flow rate [$m^3$/s]=blood layer sectional area [$m^2$]×blood flow velocity [m/s]
Blood layer sectional area=$\{(dB/2+d)^2-(dB/2)^2\}\pi[m^2]$
dB[m]: balloon diameter (calculated from an injection amount of liquid into the balloon)
d[m]: estimated blood layer thickness

3. Method of using the device of the present invention

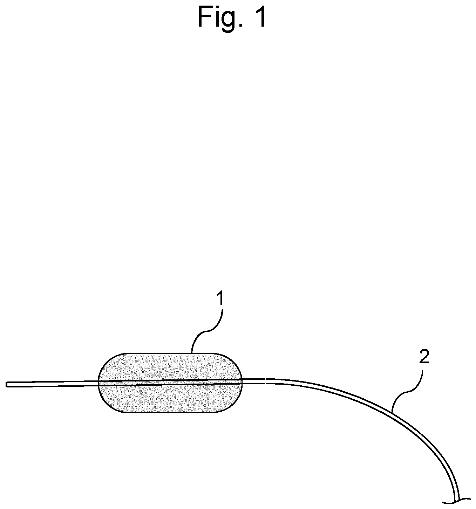

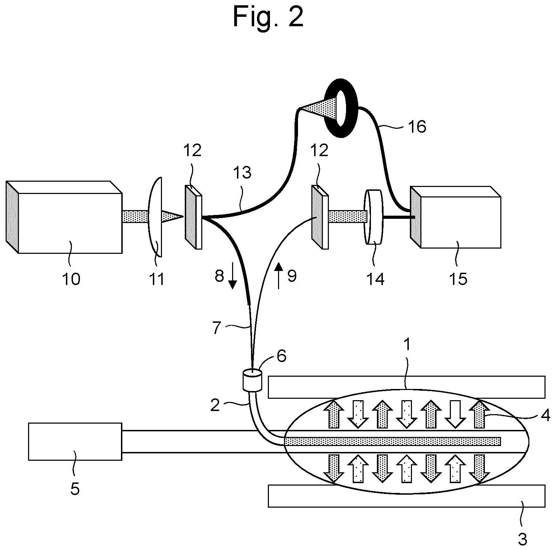

An example of a configuration of the device of the present invention is illustrated in Figure 2. An example of a configuration of the balloon catheter of the present invention is illustrated in Figure 1.
A method of using the device of the present invention is described based on Figure 2. A fiber portion of the device of the present invention has to be inserted through a sheath inserted into a blood vessel in order to insert the balloon catheter to cause the fiber portion to reach a blood vessel in which a blood flow rate is about to be monitored. Monitor feeble light is generated from a light source in Figure 2 and the light is transmitted in the fiber and radiated from the fiber distal end. The monitor light is absorbed and scattered by blood in an irradiation portion and the scattered light enters the fiber again and returns as backscattered light. A route of the returned light is changed by a beam splitter, the light is guided to a light detection part (a silicon photodiode) through an appropriate filter, and the intensity of the light is measured.
As described above, by using a plurality of wavelengths at this time, it is possible to not monitor presence or absence of a blood flow but also determine the quantity of the blood flow. Examples
The present invention is specifically described according to examples described below. However, the present invention is not limited by these examples. In the examples, an aortic blockage balloon is used as an example.

Example 1 Establishment of a trans-balloon backscattered light intensity measurement system Method

An overview of a constructed trans-balloon backscattered light intensity measurement system is illustrated in Figure 2. An aortic blockage balloon (Rescue Balloon, maximum diameter: 40 mmΦ, balloon length: 60 mm, Tokai Medical Products) was inserted into a porcine aortic lumen and balloon inflation was performed by a syringe. LED was condensed in one fiber of two-bifurcated bundle fibers (core diameter: 100 μmΦ, NA: 0.22, SI multimode, Ocean Optics), and two plastic diffused light fibers (diffusion length: 70 mm, core diameter: 250 μmΦ) connected to the fiber distal end were inserted into a central lumen of the balloon. Light irradiation was performed from one diffused light fiber and backscattered light was received from the other diffused light fiber and detected by a silicon photodiode (S2281, light reception area: 100 mm$^2$, Hamamatsu Photonics). (i) Measurement accuracy study of an experiment system using a scatterer (intralipid solution) and pig blood, (ii) measurement and accuracy study using a blood sample and a porcine aorta, and (iii) evaluation of blood flow detection performance in an *in vivo* pig model were performed.

Results

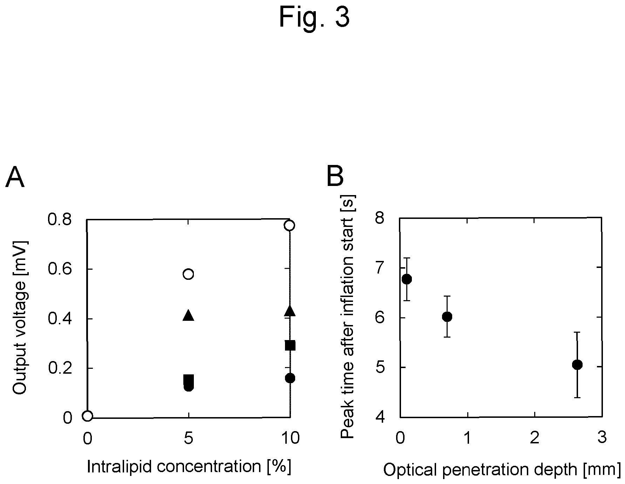

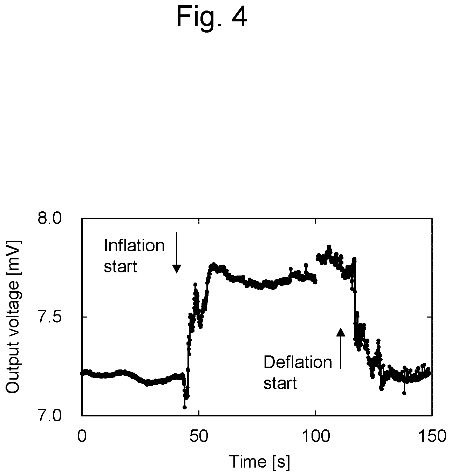

Backscattered light intensities at wavelengths in 5 and 10 (v/v)% intralipid solutions in the balloon in an inflated state are illustrated in Figure 3A. "○" (white circle) indicates red light, "A" (black triangle) indicates green light, "■" (black square) indicates cyan light, and "●" (black circle) indicates blue light. Optical penetration depth dependency of a time from an inflation start at an *ex vivo* balloon inflation time to a backscattered light intensity differential maximum value is illustrated in Figure 3B. It was successfully confirmed that the time from the inflation start to the differential maximum value was shorter as the optical penetration depth is larger. An example of a backscattered light intensity change involved in *in vivo* inflation and deflation of the REBOA balloon is illustrated in Figure 4. It was successfully confirmed that backscattered light increased after the balloon inflation even under pulsation. It was successfully confirmed that the backscattered light decreased to intensity before inflation if the balloon was deflated.

Examination

It is predicted that a change in the backscattered light intensity occurs when the optical penetration depth is equal to or smaller than the blood layer thickness between the blood vessel and the balloon. In Figure 3B, during the balloon inflation, the blood layer thickness between the blood vessel and the balloon decreases according to the inflation. Therefore, it is considered that the time from the inflation start of the balloon until the backscattered light intensity change was longer as the optical penetration depth was smaller. In Figure 4, the increase in the backscattered light from the blood vessel was successfully confirmed because the blood flow between the balloon and the blood vessel was blocked *in vivo.* Therefore, possibility of operation under pulsation was indicated. The above indicates that it is possible to prevent over inflation by radiating light from the inside of the balloon and detecting the backscattered light from the blood vessel.

Example 2 Monitoring of a blood amount using an optical penetration depth difference

(1) Estimation of a blood layer thickness

The aortic blockage balloon used in the example 1 is used and blood flow rate monitoring using an optical penetration depth difference is performed using the 395 nm light (violet), the 560 nm light (green), and the 650 nm light (red).

Absorption coefficients of the 395 nm light (violet), the 560 nm light (green), and the 650 nm light (red) are respectively 44.5 mm$^{-1}$, 12.5 mm$^{-1}$, and 0.9 mm$^{-1}$. Since the lights are less easily absorbed by blood as the absorption coefficients are smaller, optical penetration depths are larger.

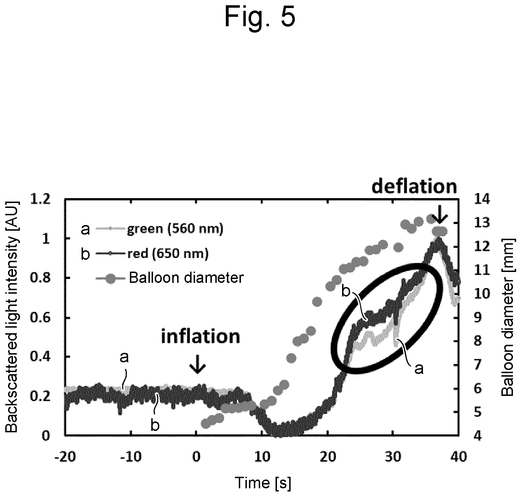

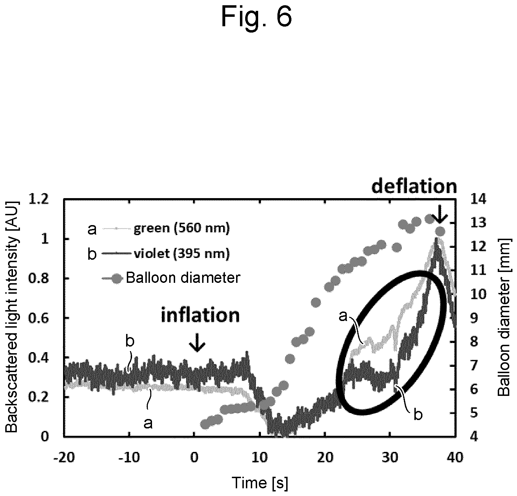

Figure 5 and Figure 6 illustrate intensities (unit AU: Arbitrary Unit) of backscattered lights of lights having wavelengths and balloon diameters in a time (the horizontal axis) from an inflation start in the case in which the 560 nm light (green) and the 650 nm light (red) are used (Figure 5) and the case in which the 395 nm light (violet) and the 560 nm light (green) are used (Figure 6). Measurements are performed until after deflation.

A difference in a diameter of the balloon at the time when each of the two color lights in the respective measurements has reached light intensity of 70% is, with the 560 nm light (green) set as a reference, 0.79 mm (the difference is larger in red) when the 560 nm light (green) and the 650 nm light (red) are used and is -1.2 mm (the difference is smaller in violet) when the 395 nm light (violet) and the 560 nm light (green) are used. In Figure 5 and Figure 6, a state in which the light has reached the light intensity of 70% is included in a portion surrounded by an ellipse.

The aortic blockage balloon in the blood vessel is inflated, backscattered light intensities at the time when lights having wavelengths are used in a process of the inflation are measured, backscattered light intensity of 70% with respect to maximum backscattered light intensity of the lights having the wavelengths is set as a threshold, and it is determined whether a measurement value in the case in which the lights having the wavelengths are used exceeds the threshold.

In Figure 7, it is indicated whether backscattered lights measured using the 395 nm light (violet), the 560 nm light (green), and the 650 nm light (red) at the time when the blood layer thickness is large to small have exceeded the threshold (indicated by "○") or have not exceeded the threshold (indicated by "×"). A schematic diagram in an upper part of Figure 7 illustrates a state in which the balloon is inserted into the blood vessel, a schematic diagram on the left illustrates a state in which the blood layer thickness is large, and a schematic diagram on the right illustrates a state in which the blood layer thickness is small. The blood layer thickness being small indicates that an inflation degree of the balloon is large and a blood flow rate is small. The blood layer thickness being large indicates that the inflation degree of the balloon is small and the blood flow rate is large. In Figure 7, the blood layer thickness is indicated in four stages of large to small (1 to 4). When the blood layer thickness is 1, since all of the 395 nm light (violet), the 560 nm light (green), and the 650 nm light (red) are easily absorbed by a substance in blood, the backscattered light intensities are small and do not exceed the threshold. When the blood layer thickness is 2, since the 650 nm light (red) having large optical penetration depth is less easily absorbed by the substance in the blood, the backscattered light intensity exceeds the threshold. However, since the 395 nm light (violet) and the 560 nm light (green), the optical penetration depths of which are not as large as the optical penetration depth of the 650 nm light (red), are easily absorbed by the substance in the blood, backscattered light intensities are small and do not exceed the threshold. When the blood layer thickness is 3, since the 650 nm light (red) and the 560 nm light (green) having large optical penetration depths are less easily absorbed by the substance in the blood, backscattered light intensities exceed the threshold. However, since the light (violet) with an optical penetration depth of 395 nm is easily absorbed by the substance in the blood, backscattered light intensity is small and exceeds the threshold. When the blood vessel layer thickness is 4, since all of the 395 nm light (violet), the 560 nm light (green), and the 650 nm light (red) are less easily absorbed by the substance in the blood, backscattered light intensities are large and exceed the threshold. In the respective measurements, the blood layer thickness can be estimated from a threshold determination pattern for determining whether the lights having the wavelengths have exceeded the threshold. When the blood layer thicknesses are 1, 2, 3, and 4, the blood layer thicknesses can be respectively estimated as >1 mm, 0.3 to 1 mm, 0.1 to 0.3 mm, and <0.1 mm.

(2) Measurement of blood flow velocity with light

An overview of a method of measuring blood flow velocity with light is illustrated in Figure 8. When the blood flow velocity is measured with light, the balloon has a lumen structure that can flush saline or the like from the distal end thereof (Figure 8A). Even if the saline is flushed without an inflation state of the balloon being changed, blood around the balloon is temporarily eliminated and backscattered light intensity changes (Figure 8B). A time required from when the saline around the balloon is flushed (Figure 8C-1 and Figure 8C-2) until when backscattered light returns to an original value again is different depending on blood flow velocity. Blood flow velocity (m/s) has to be estimated from return velocity of the backscattered light. Figure 8C-1 illustrates how the saline is flushed when a blood flow is slow and Figure 8C-2 illustrates how the saline is flushed when a blood flow is fast.

(3) Measurement of a blood flow rate

A blood flow rate can be calculated by an expression described below.

Blood flow rate $[m^3/s]$=blood layer sectional area $[m^2]×$blood flow velocity [m/s]
Blood layer sectional area=$\{(dB/2+d)^2-(dB/2)^2\}\pi[m^2]$
dB[m]: balloon diameter (calculated from an injection amount of liquid into the balloon)
d[m]: estimated blood layer thickness

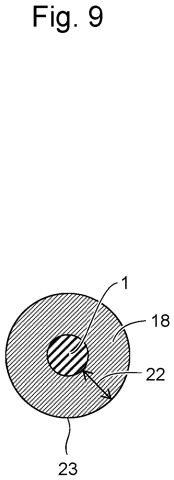

A sectional view of the blood vessel into which the balloon is inserted and the balloon and the blood layer thickness are illustrated in Figure 9.

Example 3 Study of an algorithm for estimating a blood layer thickness from backscattered light intensity

(1) Backscattered light intensity change in an *in vivo* porcine aorta

As in the example 1, backscattered lights at the time when the light (green) having the wavelength of 560 nm and the light (red) having the wavelength of 650 nm were radiated were measured using the trans-balloon backscattered light intensity measurement system. The balloon was inflated at constant speed.

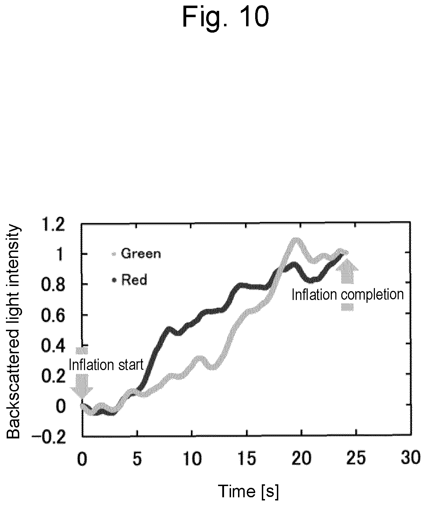

A result is illustrated in Figure 10. As illustrated in Figure 10, a change occurred in backscattered light intensity according to the balloon inflation and a difference due to a wavelength was seen in timing when the backscattered light intensity greatly changed.

(2) Study of an algorithm for estimating a blood layer thickness from the backscattered light intensity

Construction of an algorithm for estimating a blood layer thickness from backscattered light intensity data obtained in the measurement in (1) was studied. It is possible to calculate a balloon diameter (a blood layer thickness) from a time after an inflation start while keeping the balloon inflated at the constant speed.

A function represented by an expression described below was defined, fitting was performed using an elapsed time from a balloon inflation start at the time when backscattered light was detected and data of backscattered light intensity, and the coefficient $\alpha$ was optimized.

$$T = -\frac{a}{\alpha p(p-1)} log \frac{\frac{P}{1-P}}{\frac{p}{1-p}} + t \qquad (1)$$

T: time
$\alpha$: value determined for each wavelength
P: any backscattered light intensity

p: actual backscattered light intensity closest to $\frac{a}{2}$
*t:* time at the time when *P=p*

(a) Fitting in a range of 0 to 0.5 of backscattered light intensity

Fitting was performed in a range of 0 to 0.5 of backscattered light intensity using data illustrated in Figure 10. A determination coefficient was 0.98 in the case of the light (red) having the wavelength of 650 nm and was 0.92 in the case of the light (green) having the wavelength of 560 nm.

Fitting was performed in a range of 0 to 1.0 of backscattered light intensity using the data illustrated in Figure 10. The determination coefficient was 0.79 in the case of the light (red) having the wavelength of 650 nm and was 0.91 in the case of the light (green) having the wavelength of 560 nm.

The determination coefficient is a value indicating accuracy of the fitting and indicates that the accuracy is higher as the determination coefficient is closer to 1. The results of (a) and (b) described above indicate that it is preferable to estimate a blood layer thickness mainly based on data obtained using the light (green) having the wavelength of 560 nm and, in the range of 0 to 0.5 of the backscattered light intensity, a blood layer thickness can be estimated with higher accuracy by using, in combination, data obtained using the light (red) having the wavelength of 650 nm.

An elapsed time after an inflation start can be calculated by using the coefficients calculated by the optimization and substituting, in Expression (1) described above, backscattered light intensity detected by radiating the light (green) having the wavelength of 560 nm or the light having the wavelength of 650 nm. A balloon diameter is seen from the obtained elapsed time after the inflation start. A blood layer thickness can be calculated from an expression [blood layer thickness=blood vessel diameter (inner diameter)-balloon diameter] described below. Note that the balloon diameter can be learned from a time after the inflation start based on a measurement value.

Industrial Applicability

It is possible to monitor a blood flow at an aortic blockage time by using the device including the balloon catheter of the present invention.

All publications, patents, and patent applications cited in the present specification are directly incorporated in the present specification by reference.

Reference Signs List

   1 Balloon

2 Catheter
3 Porcine aorta
4 Scattered light
5 Syringe
6 Fiber insertion port
7 Scattered light fiber (diffuser length: 70 mm, core diameter: 250 μmΦ)
8 Radiation
9 Light reception
10 LED
11 Plano-convex lens
12 Fiber folder
13 Two-bifurcated bundle fiber (core diameter: 100 μmΦ each)
14 Silicon photodiode
15 AD converter
16 For incident light measurement
17 Blood elimination portion
18 Blood
19 Lumen structure
20 Saline
21 Blood flow
22 Blood layer thickness
23 Blood vessel wall

## Claims

1. A balloon catheter comprising:

    a balloon catheter including a catheter tube and a balloon provided at a distal end portion of the catheter tube;
    an optical fiber housed in the catheter tube;
    a light irradiation part disposed in the balloon and coupled to the optical fiber and a light detection part that detects radiated light; and
    a light source that generates light to be radiated, wherein
    the balloon catheter makes it possible to radiate, from the balloon, light having a wavelength that can be absorbed by a substance present in blood, detect scattered light from an inside of a blood vessel, and monitor a state of a blood flow between the balloon and a blood vessel wall over time from intensity of the detected light.

2. The balloon catheter according to claim 1, comprising an arithmetic operation part that determines presence or absence of a blood flow based on the intensity of the scattered light.

3. The balloon catheter according to claim 2, comprising a display part for displaying a state of the blood flow analyzed by the arithmetic operation part.

4. The balloon catheter according to claim 1, wherein a wavelength of the light to be radiated is 400 to 500 nm.

5. The balloon catheter according to claim 1, wherein the balloon catheter radiates, from the balloon, the light having the wavelength that can be absorbed by the substance present in the blood, the light being lights having a plurality of wavelengths with different optical penetration depths, detects scattered lights from the inside of the blood vessel of the lights having the respective wavelengths, and monitors a blood flow rate between the balloon and the blood vessel wall from intensities of the scattered lights of the lights having the respective wavelengths.

6. The balloon catheter according to claim 5, wherein the balloon catheter defines a function represented by Expression (1) or (2) described below, performs fitting using an elapsed time from a balloon inflation start at a time when backscattered light is detected and data of backscattered light intensity, determines a coefficient,

$$T = -\frac{a}{\alpha p(p-1)} log \frac{\frac{P}{1-P}}{\frac{p}{1-p}} + t \qquad (1)$$

$T$: time
$\alpha$: value determined for each wavelength
P: any backscattered light intensity

$p$: actual backscattered light intensity closest to $\frac{a}{2}$
$t:$ time at a time when $P=p$

or

$$T(P) = \frac{log \frac{P}{1-P}}{b} + c \qquad (2)$$

$T(P):$ time

P: any backscattered light intensity
b: coefficient determined for each wavelength
C: coefficient determined for each wavelength

uses the calculated coefficient and substitutes, in Expression (1) or (2) described above, backscattered light intensity detected by radiating light (green) having a wavelength of 560 nm or light having a wavelength of 650 nm to thereby calculate an elapsed time after the inflation start,
estimates a balloon diameter from the obtained time after the inflation start, and
calculates a blood layer thickness according to an expression described below [blood layer thickness=blood vessel diameter (inner diameter)-balloon diameter].

7. The balloon catheter according to claim 5 or 6, wherein the lights with the different optical penetration depths are lights having two kinds, three kinds, four kinds, five kinds, or six kinds of wavelengths among lights having wavelengths such as near infrared light, infrared light, 380 to 430 nm light (violet), 430 nm to 490 nm light (blue), 490 nm to 550 nm light (green), 550 nm to 590 nm light (yellow), 590 nm to 640 nm light (orange), and 640 to 770 nm light (red).

8. The balloon catheter according to claim 5 or 6, wherein the lights with the different optical penetration depths are at least two kinds among light having a wavelength of 475 nm, light having a wavelength of 542 nm, and light having a wavelength of 438 nm.

9. The balloon catheter according to claim 5 or 6, wherein the lights with the different optical penetration depths are two kinds of green light and red light.

10. The balloon catheter according to claim 5 or 6, wherein the lights with the different optical penetration depths are two kinds of green light having a wavelength of 560 nm and red light having a wavelength of 650 nm.

11. The balloon catheter according to any one of claims 1 to 10, wherein the balloon catheter detects the scattered light in back.

12. The balloon catheter according to any one of claims 1 to 10, wherein the catheter is a blood vessel blockage or aortic blockage catheter.

13. The balloon catheter according to claim 11, wherein the catheter is a blood vessel blockage or aortic blockage catheter.

# Fig. 1

# Fig. 2

# Fig. 3

A

B

# Fig. 4

# Fig. 5

Fig. 6

# Fig. 7

| Blood layer thickness | | Large | ⟶ | Medium | ⟶ | Small |
|---|---|---|---|---|---|---|
| Wavelength | 395 nm | × | × | × | | ○ |
| | 560 nm | × | × | | ○ | ○ |
| | 650 nm | × | ○ | | ○ | ○ |
| Estimate blood layer thickness from pattern of threshold determination | | >1 mm | 0.3-1 mm | | 0.1-0.3 mm | <0.1 mm |

# Fig. 8

A

17    1    18

2

21

B

17    1    18

2

21

C-1  Blood flow: slow

1    17    18

2

21

C-2  Blood flow: fast

1    17  18

2

21

Fig. 9

# Fig. 10

## INTERNATIONAL SEARCH REPORT

| International application No. |
|---|
| **PCT/JP2023/014708** |

### A. CLASSIFICATION OF SUBJECT MATTER

*A61M 25/10*(2013.01)i; *A61B 17/12*(2006.01)i; *A61B 5/026*(2006.01)i
FI:   A61B17/12; A61M25/10; A61B5/026 120

According to International Patent Classification (IPC) or to both national classification and IPC

### B. FIELDS SEARCHED

Minimum documentation searched (classification system followed by classification symbols)

A61M25/10; A61B17/12; A61B5/026

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | JP 2020-518329 A (THE REGENTS OF THE UNIVERSITY OF CALIFORNIA) 25 June 2020 (2020-06-25) paragraphs [0011]-[0017], [0021], [0023], [0032], [0039], [0058], fig. 1-3 | 1-4, 11-13 |
| A | | 5-10 |
| Y | US 6166806 A (TJIN, Swee Chuan) 26 December 2000 (2000-12-26) column 5, line 60 to column 6, line 24, column 6, line 66 to column 7, line 36, fig. 1-7 | 1-4, 11-13 |
| A | | 5-10 |
| Y | JP 63-109839 A (PFIZER HOSPITAL PRODUCTS GROUP INC.) 14 May 1988 (1988-05-14) p. 5, lower left column, line 15 to lower right column, last line, p. 6, upper right column, line 8 to lower left column, line 10, p. 8, upper left column, line 1 to upper right column, line 4, fig. 10, 11 | 1-4, 11-13 |
| A | | 5-10 |

☑ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **01 June 2023** | **13 June 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

International application No.

**PCT/JP2023/014708**

**C.      DOCUMENTS CONSIDERED TO BE RELEVANT**

| Category* | Citation of document, with indication, where appropriate, of the relevant passages | Relevant to claim No. |
|---|---|---|
| Y | WO 2005/079690 A1 (KEIO UNIVERSITY) 01 September 2005 (2005-09-01)<br>p. 11, line 4 to p. 13, line 15, p. 17, line 26 to p. 18, line 22, fig. 4B, 5, 9 | 2-4, 11-13 |
| A | | 5-10 |
| A | JP 2002-219130 A (OMEGA WAVE KK) 06 August 2002 (2002-08-06)<br>paragraphs [0012]-[0026], fig. 1-3 | 1 |

Form PCT/ISA/210 (second sheet) (January 2015)

**INTERNATIONAL SEARCH REPORT**
Information on patent family members

International application No.

**PCT/JP2023/014708**

| Patent document cited in search report | | | Publication date (day/month/year) | Patent family member(s) | | | Publication date (day/month/year) |
|---|---|---|---|---|---|---|---|
| JP | 2020-518329 | A | 25 June 2020 | WO 2018/195507 A1 paragraphs [0011]-[0017], [0020], [0022], [0043], [0050], [0069] US 2020/0046364 A1 CA 3060519 A1 CN 110769749 A | | | |
| US | 6166806 | A | 26 December 2000 | WO 1997/012210 A1 | | | |
| JP | 63-109839 | A | 14 May 1988 | EP 253687 A2 column 7, line 34 to column 8, line 24, column 10, lines 7-36 US 4771788 A US 4920967 A US 4991588 A | | | |
| WO | 2005/079690 | A1 | 01 September 2005 | US 2007/0167934 A1 paragraphs [0074]-[0078], [0106]-[0108] | | | |
| JP | 2002-219130 | A | 06 August 2002 | (Family: none) | | | |

Form PCT/ISA/210 (patent family annex) (January 2015)

# EP 4 509 161 A1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022064939 A

**Non-patent literature cited in the description**

- **JONKER FH et al.** *Eur J Vasc Endovasc Surg*, 2010, vol. 40, 564-71